(19) Europäisches Patentamt
European Patent Office
Office européen des brevets

(11) Publication number : **0 532 446 A1**

# (12) EUROPEAN PATENT APPLICATION

(21) Application number : **92610060.3**

(22) Date of filing : **24.08.92**

(51) Int. Cl.[5] : **A61K 31/52**

(30) Priority : **26.08.91 DK 1505/91**

(43) Date of publication of application : **17.03.93 Bulletin 93/11**

(84) Designated Contracting States : **PT**

(71) Applicant : **NOVO NORDISK A/S**
**Novo Allé**
**DK-2880 Bagsvaerd (DK)**

(72) Inventor : **Hilsted, Jannik Christian**
**Ingersvej 36**
**DK-2920 Charlottenlund (DK)**

(74) Representative : **Jorgensen, Dan et al**
**Novo Nordisk A/S, Patent Department, Novo Allé**
**DK-2880 Bagsvaerd (DK)**

## (54) Use of xanthines in the treatment or prevention of hypeoglycemia.

(57) The present invention relates to the use of a xanthine derivative of formula (I) in the manufacture of a pharmaceutical composition for counteracting hypoglycaemia in diabetic patients and to the use of a pharmaceutical composition comprising a compound of formula (I) for counteracting hypoglycaemia in diabetic patients.

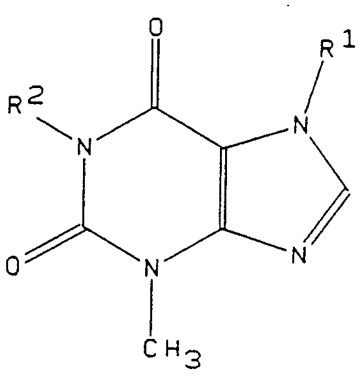

(I)

EP 0 532 446 A1

## Field of the invention

The present invention relates to the use of a xanthine derivative of formula (I) in the manufacture of a pharmaceutical composition for counteracting hypoglycaemia in diabetic patients and to the use of a pharmaceutical composition comprising a compound of formula (I) for counteracting hypoglycaemia in diabetic patients.

## Background of the invention

The normal glucose level in human whole blood is in the range 3.9 - 5.6 mmol/l. The blood glucose level rises in connection with meals and then gradually declines to approach the fasting level during the next few hours. The minimum value attained before the next rise is usually designated the nadir level. In insulin dependent diabetic patients an overdosing of insulin or a too modest food consumption may lead to hypoglycaemia, i.e. blood glucose levels lower than the normal values. If the blood glucose level declines to about 2.5 mmol/l - with individual variations - hypoglycaemia manifests itself by very unpleasant symptoms such as confusion, weakness, dizziness, headache, sweating, visual disturbance and hunger. Ultimately, if untreated, hypoglycaemia may lead to convulsions, unconsciousness, coma and even to death.

Usually hypoglycaemia can be relieved by administration of glucose or glucagon. Normally, but not always, a hypoglycaemic episode is preceded by some early warning symptoms which makes it possible for the patient to take measures against it. Throughout this specification the diabetic patients' attention to these early warning symptoms is in accordance with the common usage referred to as the "awareness".

According to a widely acknowledged, modern regimen for treating insulin dependent diabetic patients, the dosing of the insulin is adjusted so as to keep the blood glucose level as close to the normal values as possible. This is achieved by administering the daily amount of insulin as one large dose of a more or less protracted preparation to cover the basal demand, supplemented by a number of smaller doses of a rapid-acting preparation to be taken as needed e.g. in connection with meals.

According to the regimen earlier relied upon the daily amount of insulin was administered in the form of a few relatively large, fixed doses. For diabetic patients the modern regimen which tends to mimic the insulin level in non-diabetic persons in several ways improves the quality of life. Thus, it enables the patients to act more spontaneously as regards their intake of food and drink and as regards physical activity. Also the so-called long term complications seem to be less severe with a good metabolic control. Inherently, however, this modern regimen also implies that the gap between the actual blood glucose level and hypoglycaemia will in general be more narrow.

Diabetic patients are very anxious to avoid hypoglycaemic episodes, both because of the unpleasant immediate symptoms and because of the irreversible long term complications which may result from a less than optimal metabolic control. Therefore there is a strong need for the improvements provided by the present invention.

The use of medicaments based on xanthine derivatives is of long standing. Thus, caffeine has long been used as a CNS stimulant. Theobromine is a diuretic, a bronchodilator and a cardiotonic, and theophylline and many closely related compounds are smooth muscle relaxants. However, to the knowledge of the present inventor it has never been suggested to use xanthine derivatives for improving the awareness of diabetic patients who will then be in a better position to avoid hypoglycaemia or for supporting the blood glucose recovery after hypoglycaemia.

## Summary of the invention

In its broadest aspect the present invention relates to the use of a compound of the general formula (I):

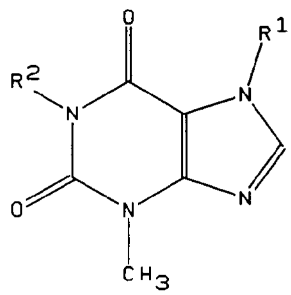

(I)

wherein $R^1$ is hydrogen, methyl, 2-hydroxypropyl, 2,3-di-hydroxypropyl, 2-nicotinoyloxyethyl, the nicotinic acid salt of 2-hydroxy-3-((2-hydroxyethyl)methylamino)propyl or a lone pair involved in salt formation with the 2-hydroxyethyltrimethylammonium ion and $R^2$ is hydrogen or methyl; a compound thereof with ethylenediamine or the pharmaceutically acceptable acid addition salts thereof in the manufacture of a pharmaceutical composition useful for counteracting hypoglycaemia in diabetic patients and to the use of a pharmaceutical composition comprising a compound of formula (I) for counteracting hypoglycaemia in diabetic patients.

According to a preferred embodiment of the present invention the compound of formula (I) is theophyllamine.

According to another preferred embodiment of the present invention the compound of formula (I) is theophylline.

According to a further preferred embodiment of the present invention the compound of formula (I) is choline theophyllinate.

According to a further preferred embodiment of the present invention the compound of formula (I) is proxyphylline.

According to a further preferred embodiment of the present invention the compound of formula (I) is glyphylline.

According to a further preferred embodiment of the present invention the compound of formula (I) is etofylline nicotinate.

According to a further preferred embodiment of the present invention the compound of formula (I) is xanthinol nicotinate.

According to a further preferred embodiment of the present invention the compound of formula (I) is caffeine.

According to a further preferred embodiment of the present invention the compound of formula (I) is theobromine.

## Detailed description of the invention

The cause of clinical diabetes is always insulin deficiency. Insulin plays a major role in the regulation of carbohydrate, lipid and protein metabolism in insulin-sensitive cells and the most familiar of insulin's effects is its ability to promote glucose transport from the blood into the tissues whenever blood glucose levels exceed normoglycaemia. The major control of insulin secretion is exerted by a feedback effect of the blood glucose level directly on the pancreas. When the level of glucose in the blood perfusing the pancreas is elevated, insulin secretion in the pancreatic venous blood is increased; when the level is normal or low, the rate of insulin secretion is low. In healthy subjects the feedback control of blood glucose on insulin secretion operates with great precision, so that blood glucose and blood insulin levels parallel each other with remarkable consistency. On the other hand patients suffering from type 1 diabetes have to take insulin and patients suffering from type 2 diabetes have to take either insulin or agents which stimulate the insulin producing β-cells of their pancreas in order i.a. to avoid hyperglycaemia. The insulin requirements depend on various factors: they rise in connection with meals, when patients gain weight, during pregnancy, during psychical stress and in the presence of infection and fever; they fall when patients lose weight and during exercise.

After careful instruction by medical staff most diabetic patients manage the day-to-day care of their disease themselves. Although the patients are usually very well trained in judging their own insulin requirements occational misjudgements some of which result in hypoglycaemia are inevitable.

Surprisingly it has now been found that compounds of formula (I) can be used for counteracting hypoglycaemia. In the present context the expression "counteracting hypoglycaemia" shall be construed to mean either "preventing the occurence of hypoglycaemic episodes" or "supporting the recovery after a hypoglycaemic episode".

As demonstrated in Table 1 and in Table 2 the nadir levels in healthy subjects as well as in diabetic patients are higher in the same person when theophyllamine is administered than with placebo. Thus, a hypoglycaemic episode is less likely to occur when theophyllamine is administered.

Similarly Table 1 and Table 2 demonstrate that blood glucose levels after 1 hour and after 2 hours are higher in the same person when theophyllamine is administered than with placebo. Thus the administration of theophyllamine supports the recovery after a hypoglycaemic episode.

Some diabetic patients who have taken theophyllamine regularly as a preventive measure have even experienced an improved awareness and may thus be able to take measures against a threatening hypoglycaemic episode at an earlier stage e.g. by the ingestion of glucose.

For counteracting hypoglycaemia in diabetic patients the preferred dose of the compound of formula (I) will generally be in the range from about 1 mg/kg body weight/day to about 6 mg/kg body weight/day, more preferred about 5 mg/kg body weight/day. The daily dose of the compound of formula (I) to be administered will depend on the specific compound employed and on the age and the condition of the patient and it is recommended that the dose of the compound of formula (I) to be given to each individual diabetic patient be determined by a physician.

The compound of formula (I) for use according to the present invention will generally be available in the form of a pharmaceutical composition. Such a composition may be in the form of a powder, a solution, or a suspension, which may or may not be divided in single dose units, or in the form of a capsule or a tablet.

The pharmaceutical composition may comprise carriers, diluents, absorption enhancers and other ingredients which are conventionally used in the art.

The route of administration may be any route which effectively transports the compound of formula (I) to its site of action, the oral or nasal route being preferred.

If a suitably protracted composition is used, the total daily dose can be given in one dose. Alternatively, the total daily dose can be subdivided in two or more smaller doses.

## Example 1

### Influence of theophyllamine on the blood glucose level in healthy subjects

Under carefully controlled conditions hypoglycaemia was induced in each of eleven healty subjects by injection of Actrapid® Human insulin, 0.15 IU/kg (available from Novo Nordisk A/S, Bagsvaerd, Denmark). The blood glucose level was monitored from just before the injection of the insulin took place until 2 hours after. The basal level, the nadir level and the level after 1 hour and 2 hours for each subject are given in Table 1 under the entry "placebo".

On a different day a study similar to the one described above was performed on the same subjects, the only difference being that in this second study theophyllamine was administered concomitantly with the insulin. The theophyllamine was administered in the form of a bolus injection of 220 mg per person given at the same time as the insulin followed by a constant rate infusion of theophyllamine of 2,5 mg/kg body weight/hour. The corresponding glucose levels are given in Table 1 under the entry "theophyllamine".

As it appears from Table 1 the nadir level was higher when theophyllamine was given concomitantly with the insulin than when insulin was given alone. Also, the glucose levels were higher 1 and 2 hours after the administration of insulin when theophylline had been given concomitantly than when insulin had been given alone.

TABLE 1

| Subject No. | 1 | 2 | 3 | 4 | 5 | 6 | 7 | 8 | 9 | 10 | 11 | MEAN | SEM |
|---|---|---|---|---|---|---|---|---|---|---|---|---|---|
| **Basal Level** | | | | | | | | | | | | | |
| Theophyllamine | 5.2 | 5.2 | 5.9 | 5.8 | 5.6 | 5.7 | 5.2 | 5.8 | 5.0 | 5.2 | 5.0 | 5.4 | 0.3 |
| Placebo | 5.2 | 5.2 | 6.2 | 5.4 | 5.4 | 5.3 | 4.9 | 5.7 | 5.5 | 5.1 | 4.5 | 5.3 | 0.4 |
| **Nadir Level** | | | | | | | | | | | | | |
| Theophyllamine | 1.5 | 2.0 | 1.9 | 1.6 | 1.7 | 2.4 | 1.5 | 2.0 | 1.3 | 1.3 | 1.4 | 1.7 | 0.5 |
| Placebo | 1.5 | 1.4 | 1.5 | 1.4 | 1.1 | 1.5 | 1.2 | 1.3 | 1.8 | 1.0 | 1.1 | 1.3 | 0.3 |
| **1 hr after insulin** | | | | | | | | | | | | | |
| Theophyllamine | 2.7 | 3.1 | 2.8 | 2.5 | 3.0 | 3.8 | 2.6 | 3.4 | 2.0 | 2.4 | 2.3 | 2.8 | 0.5 |
| Placebo | 2.2 | 2.3 | 2.9 | 2.2 | 2.1 | 2.7 | 2.4 | 2.3 | 2.3 | 2.2 | 1.8 | 2.3 | 0.3 |
| **2 hr after insulin** | | | | | | | | | | | | | |
| Theophyllamine | 3.9 | 4.3 | 4.1 | 3.5 | 4.5 | 5.4 | 4.3 | 5.1 | 3.5 | 2.6 | 3.1 | 4.0 | 0.8 |
| Placebo | 2.9 | 3.1 | 4.1 | 2.7 | 3.0 | 3.4 | 3.3 | 3.2 | 3.3 | 2.5 | 2.9 | 3.1 | 0.4 |

All levels given in mmol/1

Example 2

Influence of theophyllamine on the blood glucose level in diabetic patients

Under carefully controlled conditions hypoglycaemia was induced in each of nine diabetic patients by injection of Actrapid® Human insulin, 0.15 IU/kg (available from Novo Nordisk A/S, Bagsvaerd, Denmark). The blood glucose level was monitored from just before the injection of the insulin took place until 2 hours after. The basal level, the nadir level and the level after 1 hour and 2 hours for each subject are given in Table 2 under the entry "placebo".

On a different day a study similar to the one described above was performed on the same patients, the only difference being that in this second study theophyllamine was administered concomitantly with the insulin. The theophyllamine was administered in the form of a bolus injection of 220 mg per person followed by a constant rate infusion of theophyllamine of 2,5 mg/kg body weight/hour. The corresponding glucose levels are given in Table 2 under the entry "theophyllamine".

As it appears from Table 2 the nadir level was higher when theophyllamine was given concomitantly with the insulin than when insulin was given alone. Also, the glucose levels were higher 1 and 2 hours after the administration of insulin when theophylline had been given concomitantly than when insulin had been given alone.

TABLE 2

| Subject No. | 1 | 2 | 3 | 4 | 5 | 6 | 7 | 8 | 9 | MEAN |
|---|---|---|---|---|---|---|---|---|---|---|
| **Basal Level** | | | | | | | | | | |
| Theophyllamine | 4.8 | 4.5 | 5.5 | 4.6 | 5.6 | 4.5 | 4.7 | 6.0 | 4.9 | 5.0 |
| Placebo | 5.5 | 5.1 | 4.9 | 3.8 | 5.0 | 5.5 | 4.5 | 5.3 | 4.8 | 4.9 |
| **Nadir level** | | | | | | | | | | |
| Theophyllamine | 1.5 | 1.5 | 4.1 | 1.1 | 1.1 | 1.7 | 2.6 | 1.4 | 2.2 | 2.2 |
| Placebo | 1.6 | 1.5 | 3.0 | 1.2 | 1.0 | 1.4 | 2.6 | 1.2 | 1.9 | 1.9 |
| **1 hr after insulin** | | | | | | | | | | |
| Theophyllamine | 2.9 | 2.1 | 4.4 | 1.9 | 2.1 | 2.5 | 2.9 | 2.1 | 2.5 | 2.6 |
| Placebo | 2.5 | 2.1 | 3.3 | 1.7 | 1.6 | 2.3 | 2.8 | 1.9 | 2.2 | 2.3 |
| **2 hr after insulin** | | | | | | | | | | |
| Theophyllamine | 4.8 | 3.7 | 6.4 | 3.1 | 3.0 | 3.7 | 3.8 | 3.0 | 3.0 | 3.8 |
| Placebo | 4.4 | 3.4 | 4.7 | 2.4 | | 3.6 | 3.7 | 3.1 | 3.0 | 3.5 |

All levels given in mmol/l

## Claims

1. The use of a compound of the general formula (I):

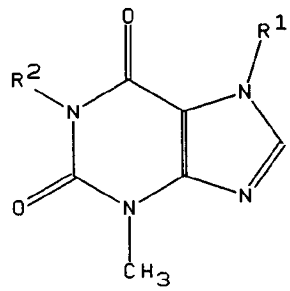

(I)

wherein $R^1$ is hydrogen, methyl, 2-hydroxypropyl, 2,3-di-hydroxypropyl, 2-nicotinoyloxyethyl, the nicotinic acid salt of 2-hydroxy-3-((2-hydroxyethyl)methylamino)propyl or a lone pair involved in salt formation with the 2-hydroxyethyltrimethylammonium ion and $R^2$ is hydrogen or methyl; a compound thereof with ethylenediamine or the pharmaceutically acceptable acid addition salts thereof in the manufacture of a pharmaceutical composition useful for counteracting hypoglycaemia in diabetic patients.

2. Use according to claim 1 characterised in that the compound of formula (I) is theophyllamine.

3. Use according to claim 1 characterised in that the compound of formula (I) is theophylline.

4. The use of a pharmaceutical composition according to any one of the preceding claims for counteracting hypoglycaemia in diabetic patients.

5. Any novel feature or combination of features as herein described.

European Patent Office

# PARTIAL EUROPEAN SEARCH REPORT

which under Rule 45 of the European Patent Convention shall be considered, for the purposes of subsequent proceedings, as the European search report

Application Number
EP 92 61 0060
Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| X | MED KLIN, VOL. 64, NO. 22, PAGES 1042-1045, 1969,<br>J. HAHN ET AL. 'Beeinflussung der Therapie und Einstellung von Diabetikern durch Xanthinnikotinal.'<br>* page 1043 *<br>* page 1045 *<br>--- | 1,4 | A61K31/52 |
| X | J.E.F. REYNOLDS & A.B. PRASAD (ED,) 'Martindale, the Extra Pharmacopoeia.' 1982 , THE PHARMACEUTICAL PRESS , LONDON, GB<br>* page 340 - page 341 *<br>--- | 1,4 | |
| X | DIABETES, VOL. 24, NO. 3, PAGES 249-256, 1975, US<br>L. SACCA ET AL. 'Effects of theophylline on glucose kinetics in normal and sympathectomized rats.'<br>* page 253 - page 255; figures 1-2 *<br>---<br>-/-- | 1-4 | |

| TECHNICAL FIELDS SEARCHED (Int. Cl. 5) |
|---|
| A61K |

## INCOMPLETE SEARCH

The Search Division considers that the present European patent application does not comply with the provisions of the European Patent Convention to such an extent that it is not possible to carry out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 09 DECEMBER 1992 | DULLAART A.W.M. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04E07)

European Patent Office

# PARTIAL EUROPEAN SEARCH REPORT

Application Number
EP 92 61 0060
Page 2

| DOCUMENTS CONSIDERED TO BE RELEVANT | | | CLASSIFICATION OF THE APPLICATION (Int. Cl. 5) |
|---|---|---|---|
| Category | Citation of document with indication, where appropriate, of relevant passages | Relevant to claim | |
| X | PEDIATRIE, VOL. 38, NO. 5, PAGES 303-308, 1983, L. SANN ET AL. 'Evolution de la glycemie apres arret d'une perfusion glucosee chez l'enfant de petit poids de naissance.' * page 307 * --- | 1-4 | |
| X | METABOLISM, VOL. 34, NO. 1, PAGES 92-96, January 1985, J.D. WILSON ET AL. 'Pancreatic islet allograft function in nonimmunosuppressed conscious mice.' * page 93 * ----- | 1-4 | |
| | | | TECHNICAL FIELDS SEARCHED (Int. Cl. 5) |

EPO FORM 1503 03.82 (P04E10)

EP 92 61 0060 -C-

INCOMPLETE SEARCH

Claim not searched : 5

Reason : Rule 29(6) EPC.